# EUROPÄISCHE PATENTANMELDUNG

(11) **EP 2 842 606 A1**
(43) Veröffentlichungstag der Anmeldung: **04.03.2015**
(21) Anmeldenummer: 13181661.3
(22) Anmeldetag: 26.08.2013
(51) Int. Cl.: A61Q 19/00, A61K 8/98, A61K 8/02, A61K 8/73

(54) **Kosmetisches Yoghurtmaskenset und Verfahren zur Herstellung einer Yoghurtmaske**

(71) Anmelder: PM-International AG, 1618 Luxembourg (LU)
(72) Erfinder: Golz-Berner, Karin, MC-9800 Monaco (MC); Meinhardt, Horst, DE-56249 Herschbach (DE); Sorg, Rolf, L-5444 Schengen (LU)
(74) Vertreter: Gulde & Partner

(57) **Zusammenfassung**

Die Erfindung betrifft ein kosmetisches Yoghurtmaskenset, mit dem sich Masken für bestimmte Körperteile wie Hals, Gesicht, Dekolleté herstellen lassen.

Das Yoghurtmaskenset besteht aus einem portionierten Yoghurtpulver aus verschiedenen probiotischen Yoghurtkulturen im Trockenzustand sowie eiweißangereichertem Süßmolkenpulver, weiterhin aus einem pulverförmigen Verdickungsmittel in Form einer modifizierten Maisstärke, einem thermoisolierenden Zubereitungsbehälter, einem händischen Rührgerät sowie einer Zubereitungsanleitung mit Hinweis auf das Verhältnis Yoghurtpulver, Milch und Verdickungsmittel und die Reihenfolge des Vermischens.

Ein entsprechendes Herstellungsverfahren wird beschrieben. Die Yoghurtmaske führt zu einer einzigartigen Konsistenz, läßt sich sehr anwenderfreundlich auftragen und ist lagerstabil im angerührten Zustand.

## Beschreibung

Die Erfindung betrifft ein kosmetisches Yoghurtmaskenset, mit dem sich Masken für bestimmte Körperteile wie Hals, Gesicht, Dekolleté herstellen lassen.

Die Selbstherstellung von kosmetischen Masken wird im Interne sowohl von einzelnen Anwendern als auch von Firmen zum Teil sehr detailliert beschrieben. Die Rezepturen gehen von "3 Eßlöffel Quark, 2 Eßlöffel Joghurt, 15 Tropfen Olivenöl, 1 Eigelb und Vermengen zu einer Paste" oder so ähnlich bis zu Hinweisen, daß in Kosmetikstudios je nach Konsistenz eine (fertige) Maske mit dem Pinsel auf die Haut aufgetragen wird. Die Einstellung der Konsistenz wird auch von den Fachleuten nicht näher beschrieben und erfolgt meist nach der "trial-and-error"-Methode.

In der DE 102007039229 A1 wird ein wasser- und wirkstoffhaltiges Gel beansprucht, das mehrere Gelbildner wie Kelcogel F, Kelcogel LT1 00 und Jaguar enthält, und das zu einem flexiblen Erzeugnis z. B. als Gesichtsmaske gegossen wird.

In einem Abstract für die KR 20070117311 (A) wird eine kosmetische Maske mit Infrarot-emittierenden Pulverbestandteilen (15-25 Gew.-%) aus Löß, Jade, sowie Alginatpulver (60-80 Gew.-%) und anderen Nährstoffen wie Honig, Yoghurt, Milch offenbart. Die Konsistenz wird offensichtlich stark durch die mineralischen Bestandteile bestimmt.

Der Erfindung liegt die Aufgabe zugrunde, ein Set für eine kosmetische Maske bereitzustellen, mit dessen Hilfe ein Anwender eine Yoghurtmaske bereiten kann, die eine Vielzahl von Hautnährstoffen zur Verfügung stellt, lagerstabil ist und eine breiartige, halbflüssige Konsistenz hat, die ein Auftragen mit den Fingern gestattet, ohne daß die Maskenmasse leicht von den Fingern abgleitet.

Eine weitere Aufgabe der Erfindung besteht in der Bereitstellung eines Verfahrens zur Herstellung einer Yoghurtmaske.

Das erfindungsgemäße Yoghurtmaskenset umfaßt
a) ein portioniertes, gefriergetrocknetes Yoghurtpulver aus einem Gemisch von probiotischen Kulturen, wobei dies die Kulturen Lactobacillus acidophilus, Bifidobacterium lactis, Streptococcus thermophilus und Lactobacillus delbrückii sind, sowie ein eiweißangereichertes Süßmolkenpulver
b) ein portionierten, pulverförmiges Verdickungsmittel in Form einer modifizierten Maisstärke
c) einen thermoisolierenden Zubereitungsbehälter
d) ein händisches Rührgerät und
e) eine Zubereitungsanleitung mit Hinweis auf das Verhältnis Yoghurtpulver, Milch und Verdickungsmittel und die Reihenfolge des Vermischens.

Die Bestandteile a) und b) liegen jeweils im Trockenzustand vor.

Das Yoghurtpulver kann zusätzlich Inulin, Niacin oder ein Gemisch davon enthalten. Auch Aroma- oder Duftstoffe können in dem Yoghurtpulver enthalten sein.

Vorteilhaft ist das Gemisch der probiotischen Kulturen in einer Folienabpackung eingeschlossen, die den Licht- und Sauerstoffdurchtritt hemmt und dadurch die Aktivität der Kulturen aufrechterhält.

Der Begriff "portioniert" bedeutet, daß die Menge an Yoghurtpulver in einem bestimmten Verhältnis zur Menge an Verdickungsmittel steht. Das Verhältnis liegt vorteilhaft im Bereich von 1 : 0,5 - 3.

Als Verdickungsmittel wird vorzugsweise eine Maisstärke mit der INCI-Bezeichnung Hydroxypropyl Starch Phosphate eingesetzt.

Es wurde gefunden, daß andere Verdickungsmittel sowohl einzeln als auch im Gemisch nicht zugleich zu der angestrebten brei-artigen Konsistenz und dem ebenfalls angestrebten sehr angenehmen Hautgefühl führen, sondern zu leicht von den Fingern abgleiten und damit das Ziel, ein komfortables Auftragen auf die Haut mit gleichzeitigem weichen Hautfeeling zu ermöglichen, nicht erreichen.

Überraschenderweise führen solche bekannten Verdickungsmittel wie Guar Gum, Gellan Gum oder Carbomer im Gemisch mit gereiftem Yoghurt auf Kuhmilchbasis zu keiner homogenen Masse, sondern sie verringern sogar die bestehende Viskosität der Masse, so daß diese nicht in der gewünschten Form auftragbar wird.

Der thermoisolierende Zubereitungsbehälter besteht vorteilhaft aus einem Innengefäß und einem Mantelgefäß, das das lnnengefäß umgibt. Das Innengefäß ist für das Vermischen von Milch bei Umgebungstemperatur (15-25°C) mit dem Yoghurtpulver bestimmt. Das Mantelgefäß ist für die Befüllung mit einer bestimmten Menge (je nach Füllhöhe im Innengefäß) Wasser von 80-100°C bestimmt.

Das Innengefäß hat vorteilhaft etwa 1,1 bis 1,3 I Volumen und ist vorzugsweise zylinderförmig.

Das Mantelgefäß kann noch eine zusätzliche Isolierung nach außen hin aufweisen, z. B. eine dünne Schaumpolystyrolschicht.

Nach Abschluß der Reifungsphase des mit Milch angerührten Yoghurts erfolgt der Vermischungsvorgang von Yoghurt und Verdickungsmittel. Das dafür eingesetzte händische Rührgerät ist vorteilhaft ein Rührstab, der gegebenenfalls Querschnittsvergrößerungen im mittleren bis unteren Teil aufweisen kann, um die Rührfläche zu vergrößern. Es kann auch ein Spatel eingesetzt werden.

Das Rührgerät ist deshalb ein "händisches", d. h. von Hand zu führendes und ohne elektrischen Antrieb, da damit eher ein sehr langsames Rühren realisiert wird, um die erforderliche Konsistenz der Maske zu gewährleisten und die sich mit dem Verdikkungsmittel ausbildenden Strukturen nicht zu zerstören.

In der erfindungsgemäßen Zubereitungsanleitung wird das Mischungsverhältnis von Yoghurt und Verdickungsmittel festgelegt. Dieses Verhältnis liegt im Bereich von 3,5 - 4,5 Teile gereifter Yoghurtmasse zu 1 Teil pulverförmigem Verdickungsmittel, insbesondere im Verhältnis 3,7 - 4,2 : 1 (Gewichtsteile).

Weiterhin ist festgelegt, daß - anders als bei der üblichen Verfahrensweise - das Verdickungsmittel in den Yoghurt unter langsamer Zudosierung des Verdickungsmittels und bei langsamem Rühren zugegeben wird.

Die übliche Verfahrensweise für die Pigmentvermischung mit viskosen Massen erfolgt wie in der keramischen Industrie bei Pigment und Schlicker in der Weise, daß das Pigment - dies ist hier die modifizierte Stärke - vorgelegt und der Schlicker - hier die Yoghurtmasse - damit verrührt wird, um Homogenität zu erhalten. Überraschend führt diese Verfahrensweise hier nicht zum Erfolg, sondern die Masse wird nicht homogen. Erfindungsgemäß wird die Stärke in einem sehr langsamen und empfindlichen Rührvorgang mit der Yoghurtmasse bei nacheinanderfolgendem Einstreuen des Stärkepulvers in die Yoghurtmasse hinzugegeben, und man erhält eine homogene Mischung.

"Langsam" bedeutet, 60-150 Rührumdrehungen pro Minute bis zur homogenen Verteilung, was eine Durchführung des Rührvorgangs über wenigstens 2 Minuten erfordert, so daß keine Reste des pulverförmigen Verdickungsmittels mehr sichtbar sind.

Die Zusammensetzung der Yoghurtmaske mit dem probiotischen Milchsäurebakterien führt in erster Linie zu einer intensiven Neutralisierung gesundheitsschädlicher Mikroorganismen auf der zu behandelnden Haut sowie zur Bekämpfung freier Radikale, die durch Umwelteinflüsse wie UV-Einstrahlung, Smog, Zigarettenrauch usw. besonders auf den unbedeckten Partien menschlicher Haut wie Gesicht, Hals, Dekolleté zu finden sind. Weiterhin werden wichtige Nährstoffe wie Vitamine, Spurenelemente usw. direkt und intensiv für einen längeren Zeitraum zugeführt. Dazu kommen die in der Milch vorhandenen pflegenden Bestandteile.

Ein bevorzugtes probiotisches Gemisch von Milchsäurebakterien ist das in der EP 1516537 B1 beschriebene Gemisch, insbesondere ein Gemisch von Lactobacillus acidophilus La-5, Bifidobacterium lactis Bb-12, Streptococcus thermophilus und Lactobacillus delbrückii susps. Bulgaricus.

Das zugesetzte Inulin als Prebiotic ist ein Hilfs- und Nährstoff für die Förderung der probiotischen Kulturen und beeinflußt die Aktivität und das Wachstum dieser. Auch Lactose kann für diesen Zweck eingesetzt werden.

Ein kapillardilativer Wirkstoff wie Niacin fördert eine verbesserte Sauerstoffaufnahme und Stoflwechselaustausch im Mikrozirkulationsbereich. Der Niacinanteil im Pulver liegt bei 20-200 mg pro Liter fertiger Yoghurtmaske.

Ein weiterer wesentlicher Vorteil der Erfindung ist die Verbesserung der Lagerbeständigkeit der mit dem Verdickungsmittel angerührten Yoghurtmasse. Die spezielle Yoghurtzusammensetzung mit dem speziellen Verdickungsmittel führt insgesamt zu einer Masse, die bei einer üblichen Lagerzeit im Kühlschrank bei 6-9°C über eine Woche nur sehr geringe Absetzerscheinungen von Wasser zeigt und daher auch nach dieser Lagerzeit ohne weiteren Rührvorgang vom Anwender auf die Haut aufgetragen werden kann.

Ein weiterer wesentlicher Vorteil der Erfindung ist die Bereitstellung eines Sets, das bei anleitungsgemäßer Zubereitung der Yoghurtmaske zu einer brei-artigen Konsistenz führt. Diese Konsistenz erlaubt es, eine bestimmte Menge der Masse z. B. mit einem Löffel zu entnehmen, auf die Finger zu übertragen und mit den Fingern bis zur gewünschten Schichtdicke auf der Haut zu verteilen, ohne daß die Masse leicht von den Fingern abfließt oder abgleitet. Sie hat ein bestimmtes Haftvermögen, das die Verteilung leicht macht und unerwünschtes Abgleiten oder -fließen vollständig vermeidet. Diese bisher einzigartige Eigenschaft bei Yoghurtmasken ist die Folge der speziellen Zusammensetzung und der spezifischen Zubereitung.

Die Erfindung betrifft daher auch ein Verfahren zur Herstellung einer Yoghurtmaske, das dadurch gekennzeichnet ist, daß ein portionierte, gefriergetrocknetes Yoghurtpulver aus einem Gemisch von probiotischen Kulturen, wobei dies die Kulturen Lactobacillus acidophilus, Bifidobacterium lactis, Streptococcus thermophilus und Lactobacillus delbrückii sind, sowie ein eiweißangereichertes Süßmolkenpulver mit Milch angerührt und bei erhöhter Temperatur im Bereich von 32-38°C einem Reifungsvorgang über 8-10 Stunden unterworfen wird, danach der ausgereiften flüssigen Yoghurtmasse ein Verdickungsmittel in Form einer modifizierten Maisstärke im Verhältnis 3,5-4,5 Teile Yoghurtmasse zu 1 Teil Verdickungsmittel unter Rühren von Hand mit einem Rührstab mit 60-150 Rührumdrehungen pro Minute nach und nach zugesetzt und die homogen erscheinende Yoghurtmasse 50 bis 70 Minuten stehengelassen wird.

Ein bevorzugtes Mischungsverhältnis ist der Einsatz von 48-53 g Yoghurtpulver mit der oben beschriebenen Zusammensetzung und einem Liter Milch, vorzugsweise H-Kuhmilch. Von der gereiften Yoghurtmasse können dann z. B. 100 g abgenommen und mit 22-28 g der modifizierten Maisstärke vermischt werden.

Der restliche Teil der Yoghurtmasse kann nach Lagerung im Kühlschrank später verwendet oder als Nahrungsmittel eingesetzt werden.

"Rührumdrehungen" bedeutet vollständige Kreise mit einem Rührstab.

Die Anzahl der probiotischen Keime liegt insgesamt vorteilhaft bei über 10 Millionen pro Gramm, bevorzugt über 120 Mio/g, jeweils bezogen auf die ausgereifte Yoghurtmasse.

Die Erfindung wird nachstehend durch Beispiele näher erläutert.

### Beispiel 1 Set

Es wird ein kosmetisches Yoghurtmaskenset zusammengestellt mit den folgenden Bestandteilen
1. Eine licht- und sauerstoffdichte Folienabpackung als Sachet oder Beutel, enthaltend ca. 50 g Yoghurtpulver, davon ca. 100 mg Niacin
   Das Yoghurtpulver besteht aus einem Gemisch gefriergetrockneter, probiotischer Yoghurtkulturen mit den Bestandteilen Lactobacillus acidophilus La-5, Bifidobacterium lactis Bb-12, Streptococcus thermophilus und Lactobacillus delbrückii susps. Bulgaricus sowie den weiteren Bestandteilen eiweißangereichertes Süßmolkenpulver, Inulin, Niacin und Aroma-/Duftstoffe.
2. eine Folienabpackung, enthaltend 100 g Hydroxypropyl Starch Phosphate (Structure ® XL Starch von Akzo Nobel, Sempach-Station, Schweiz)
3. ein zylinderförmiger Zubereitungsbehälter mit einem Innengefäß aus geschmacksneutralem lebensmittelgerechten Kunststoff mit einem Volumen von 1,2 I und einem Mantelgefäß mit einer Zugießöffnung für heißes Wasser am Mantelgefäß
4. ein Rührstab aus einem geschmacksneutralen, lebensmittelgerechten Kunststoff mit einer über die Zylinderhöhe des Innenbehälters hinausgehenden Länge
5. eine Zubereitungsanleitung mit der Festlegung, daß die portionierte Menge an Yoghurtpulver, z. B. 50 g mit 1 I Milch in dem Innenbehälter zu vermischen ist, der Mantelbehälter mit heißem Waser zu befüllen ist und die Milch kurz zu rühren ist, für die zugedeckten Behälter eine Reifungszeit von 8-10 Stunden einzuhalten ist. Danach ist die Stärke als Verdickungsmittel mit der gewünschten Teilmenge der gereiften Yoghurtmasse im Verhältnis 1 : 3,5-4,5 zu vermischen. Bei einer Teilmenge von 100 g gereifter Yoghurtmasse werden somit ca. 22-28 g Verdickungsmittel zugegeben. Dies erfolgt unter Rühren mit 60-150 Ulmin mit dem Rührstab in die Yoghurtmasse bis zur homogenen Verteilung, und die Yoghurtmasse ist 50-70 Minuten stehenzulassen, bevor eine Anwendung als Maske erfolgt.

Die entsprechenden Kunststoffe sollen geschmacksneutral und lebensmittelgerecht sein, da es naheliegend ist, daß Reste der Yoghurtmasse von dem Anwender/der Anwenderin verzehrt werden. Geeignete Kunststoffe sind PE, PP, PA6, PET, PVDF, PEEK, PSU, PES usw. Es kann allerdings auch ein Holzspatel eingesetzt werden.

### Beispiel 2 Verfahren

Es werden etwa 50 g eines Yoghurtpulvers mit der Zusammensetzung wie im Beispiel 1 beschrieben mit 1 I H-Milch bei Umgebungstemperatur vermischt und in das Innenteil eines Reifungsbehälters gegeben. Der Mantelteil des Reifungsbehälters, der den Innenteil umhüllt, wird mit heißem Wasser etwa bis zur Füllhöhe der Milch im Innenteil befüllt, und die Milch im Innenteil wird zwecks besserer Temperaturverteilung gerührt. Der zugedeckte Reifungsbehälter wird 9 Stunden in Ruhe stehengelassen.

Danach werden mit einem Rührstab von Hand z. B. 24 g des Verdickungsmittels zu 100 g gereifter Yoghurtmasse nacheinander zugegeben und mit einer Geschwindigkeit von ca. 90 Rührrunden pro Minute eingerührt bis zu einer homogenen Verteilung, z. B. für 3 Minuten.

Nach dem Homogenisieren wird die Yoghurtmasse 55 Minuten stehengelassen.

Die Masse mit brei-artiger Konsistenz kann dann mit einem sauberen Gerät, z. B. einem Löffel, entnommen, auf die Finger übertragen und mit diesen auf das Gesicht des Anwenders als kosmetische Yoghurtmaske unter Aussparung von Mund, Augen und Ohren in gewünschter Dicke aufgetragen werden.

Die Masse fließt nicht von den Fingern, sondern hat auch in größerer Menge z. B. bis zu 0,5 bis 2 cm Dicke einen guten Zusammenhalt und läßt sich damit komfortabel auf die Gesichtsfläche übertragen.

Nach entsprechender Verweilzeit auf dem Gesicht läßt sich die Maske leicht mit klarem Wasser abspülen und führt zu einem ungemein weichen Hautgefühl und frischem Teint als Folge der Zuführung von Nährstoffen und Anregung der Mikrozirkulation der Haut.

### Beispiel 3 Vergleich

Es wurden 50 g Yaghurtpulver ( My-Yo-Bio-Joghurtpulver) mit warmer Milch (1 I) verrührt und in einem Reifungsbehälter bei 30-36 °C für 8 Stunden stehen gelassen.

100 g der erhaltenen gereiften Yoghurtmasse wurden in üblicher Weise zu einem Verdickungsmittel gegeben und mit diesem mit einem Handmixer mit 400-500 U/min verrührt.
Probe A: Verdickungsmittel 50 g Xanthan Gum
Probe B: Verdickungsmittel 30 g Dextrin
Probe C: Verdickungsmittel 30 g Gellan Gum
Probe D: Verdickungsmittel 50 g Carbomer.

Die Proben wurden danach 1 h stehen gelassen. Von jeder Probe wurde nacheinander eine Teilmenge mit einem Löffel entnommen und auf die Finger von 4 Probandinnen gegeben, die jeweils eine Gesichtsmaske davon auf ihr Gesicht auftragen sollten. Die Test wurden dreimal wiederholt.

*Probe A*: Die Yoghurtmasse war bei allen drei Tests zu flüssig und tropfte von den Fingern. Nachträglicher Zusatz von 10 g Xanthan Gum führte zu einer glitschigen Masse, die ebenfalls zu leicht von den Fingern lief. Die übertragenen Reste der Masse wurden beim Verteilen auf der Gesichtshaut als "sehr sumpf" eingeschätzt.

*Probe B*: Die Yoghurtmaske war bei allen drei Tests zu flüssig und tropfte von den Fingern. Nachträglicher Zusatz von 10 g Dextrin führte zu einer Masse, die zu dick war. Zwischenwerte ließen sich kaum einstellen. Die übertragene Masse wurde beim Verteilen auf der Gesichtshaut als 3,wenig gleitfähig" oingesehätzt.

*Probe C*: Die Yoghurtmasse war bei allen drei Tests zu flüssig und glitt zu leicht von den Fingern ab. Nachträglicher Zusatz von 10 g Gellan Gum führte zu einer nicht ausreichenden Verbesserung. Die übertragenen Reste der Masse wurden beim Verteilen auf der Gesichtshaut als "gut" eingeschätzt.

*Probe D*: Die Yoghurtmasse war bei allen drei Tests zu flüssig und tropfte von den Fingern. Nachträglicher Zusatz von 10 g Carbomer führte zu einer noch immer zu stark gleitenden Masse, die zu leicht von den Fingern lief. Bei der Übertragung der Reste der Masse und Verteilen auf der Gesichtshaut wurde von den Probandinnen ein "gutes Gefühl" konstatiert.

Eine weitere Probe E entsprach Beispiel 2 der Erfindung.

*Probe E*: Die Yoghurtmasse lief nach Übertragung auf die Finger einer fünften Probandin nicht ab, sondern erlaubt eine Schichtdicke von ca. 1 cm auf den Fingern ohne Abtropfen auf das Gesicht der Probandin zu übertragen. Hervorgehoben wurde das sehr angenehme Hautgefühl bei der Verteilung der Masse auf der Gesichtshaut.

### Beispiel 4 Lagertest

Die Proben A bis E wurden in einer Menge von jeweils 150 g in ein Becherglas gegeben und für 6 Tage im Kühlschrank bei 8-10 °C gelagert.

Nach dieser Zeit zeigten die Proben A, C und D eine abgesetzte wäßrige Schicht von ca. 3 mm. Probe B zeigte eine wäßrige Schicht von ca. 5 mm. Probe E zeigte eine abgesetzte Schicht von nur 0,5 mm und damit eine deutlich bessere Lagerbeständigkeit.

## Patentansprüche

1. Kosmetisches Yoghurtmaskenset, **gekennzeichnet durch**
a) ein portioniertes Yoghurtpulver, umfassend ein Gemisch der probiotischen Yoghurtkulturen Lactobacillus acidophilus, Bifidobacterium lactis, Streptococcus thermophilus und Lactobacillus delbrückii susps. Bulgaricus im Trockenzustand sowie eiweißangereichertes Süßmolkenpulver,
b) ein pulverförmiges Verdickungsmittel in Form einer modifizierten Maisstärke
c) einen thermoisolierenden Zubereitungsbehälter
d) ein händisches Rührgerät und
e) eine Zubereitungsanleitung mit Hinweis auf das Verhältnis Yoghurtpulver, Milch und Verdickungsmittel und die Reihenfolge des Vermischens.

2. Yoghurtmaskenset nach Anspruch 1, worin das Yoghurtpulver Inulin oder Niacin oder ein Gemisch davon umfaßt.

3. Yoghurtmaskenset nach Anspruch 1 oder 2, worin die modifizierte Maisstärke Hydroxypropyl Starch Phosphate ist.

4. Yoghurtmaskenset nach einem der Ansprüche 1 bis 3, worin das Verhältnis des portionierten Yoghurtpulvers zu dem portionierten Verdickungsmittel im Bereich von 3,5 bis 4,5 Teile mit Milch gereifter Yoghurtmasse zu 1 Teil pulver-förmigem Verdickungsmittel liegt.

5. Yoghurtmaskenset nach einem der Ansprüche 1 bis 4, worin der Zubereitungsbehälter aus einem Innenbehälter für das Gemisch aus Yoghurtpulver und Milch und einem Mantelbehälter für eingefülltes Heißwasser besteht.

6. Yoghurtmaskenset nach einem der Ansprüche 1 bis 5, worin das Rührgerät ein gegebenenfalls mit Querschnittsverdickungen versehener Rührstab ist.

7. Yoghurtmaskenset nach einem der Ansprüche 1 bis 6, worin in der Zubereitungsanleitung die Vermischungsfolge von vorgelegter gereifter Yoghurtmasse und zugesetztem Verdickungsmittel festgelegt ist.

8. Verfahren zur Herstellung einer Yoghurtmaske, **dadurch gekennzeichnet, daß** ein portioniertes, gefriergetrocknetes Yoghurtpulver aus einem Gemisch von probiotischen Kulturen, wobei dies die Kulturen Lactobacillus acidophilus, Bifidobacterium lactis, Streptococcus thermophilus und Lactobacillus dolbrückü sind, sowie ein eiweißangereichertes Süßmolkenpulver mit Milch angeführt und bei erhöhter Temperatur im Bereich von 32-38°C einem Reifungsvorgang über 8-10 Stunden unterworfen wird, danach der ausgereiften flüssigen Yoghurtmasse ein Verdickungsmittel in Form einer modifizierten Maisstärke im Verhältnis 3,5-4,5 Teile Yoghurtmasse zu 1 Teil pulverförmigem Verdickungsmittel unter Rühren von Hand mit einem Rührstab mit 60-150 Rührumdrehungen pro Minute nach und nach zugesetzt und die homogen erscheinende Yoghurtmasse 50 bis 70 Minuten stehengelassen wird.

9. Verfahren nach Anspruch 8, worin als modifizierte Maisstärke Hydroxypropyl Starch Phosphate zugesetzt wird.

10. Verfahren nach Anspruch 8, worin 50 g Yoghurtpulver mit 1 I Milch angerührt und bei 34-37°C über 8-10 Stunden zum Reifen stehengelassen werden, danach für 100 g Yoghurtmasse 22 bis 28 g Hydroxypropyl Starch Phosphate mit dem Rührstab in den gereiften Yoghurt bei 60-150 Rührumdrehungen pro Minute, vorzugsweise 80-120 U/min eingerührt und dann 50-60 Min. stehengelassen wird.
